# FASCICULE DE BREVET EUROPEEN

(11) **EP 0 737 451 B1**
(45) Date de publication et mention de la délivrance du brevet: **10.09.2003**
(21) Numéro de dépôt: 96400783.5
(22) Date de dépôt: 11.04.1996
(51) Int. Cl.: A61F 2/01

(54) **Dispositif médical intraluminal, tel que filtre sanguin**
Intraluminale medische Vorrichtung, insbesondere Blutfilter
Intraluminal medical device, such as blood filter

(30) Priorité: 14.04.1995 EP 95400850
(43) Date de publication de la demande: 16.10.1996
(73) Titulaire: B. Braun Medical SAS, 92100 Boulogne Billancourt (FR)
(72) Inventeur: Chevillon, Gérard, 92120 Montrouge (FR); Nadal, Guy, 86000 Poitiers (FR)
(74) Mandataire: Lerner, François

(56) Documents cités:
- EP-A- 0 348 295
- EP-A- 0 582 493
- FR-A- 2 573 646
- FR-A- 2 666 980
- FR-A- 2 699 810
- FR-A- 2 717 069
- US-A- 5 344 427

## Description

L'invention concerne d'une manière générale un ensemble médical adapté pour être introduit dans un conduit anatomique du corps d'un patient, comprenant un dispositif intraluminal et des moyens d'implantation ou de retrait du dispositif, selon le préambule de la revendication indépendante 1.

FR-A-2 666 980 illustre un tel ensemble médical.

Or, des problèmes se posent lorsque l'on souhaite pouvoir implanter temporairement des filtres et plus généralement des dispositifs médicaux intraluminaux du type précité, en offrant donc au praticien la possibilité de retirer le dispositif après une période d'implantation plus ou moins longue (pouvant aller jusqu'à quelques semaines, dans le cas des filtres sanguins existants).

Le problème que soulève la présente invention est en effet lié aux exigences pratiques d'utilisation de tels dispositifs qui doivent prendre en compte à la fois :
- un problème de développement cellulaire autour du tronçon des pattes venant s'appliquer contre le conduit récepteur, lequel développement ne doit pas empêcher le retrait du dispositif en fin d'implantation temporaire,
- un problème de dimensionnement en particulier radial du dispositif, compte tenu de ce que l'on souhaite pouvoir l'implanter par des gaines de plus petits diamètres possible,
- et un souci complémentaire concernant la flexibilité que l'on souhaite assurer aux pattes, tout particulièrement sur la longueur de leur dit "second tronçon", de manière à favoriser le passage du dispositif entre son premier diamètre réduit et son second diamètre élargi, avec la souplesse et l'élasticité voulues.

Or, toutes ces exigences se sont avérées jusqu'à présent difficiles à concilier. Et aucun art antérieur connu du demandeur ne les a, à ce jour, pris en compte ensemble.

C'est pourquoi, dans le but d'apporter une solution satisfaisante à cela, l'invention propose que :
- le premier tronçon de certaines au moins des pattes du dispositif présente une surface aplatie essentiellement pleine d'appui contre la paroi du conduit,
- sensiblement perpendiculairement à l'axe principal et dans l'état replié du dispositif, ce premier tronçon présente une dimension maximale supérieure à celle du second tronçon, et
- le premier tronçon est dépourvu de moyens d'accrochage à la paroi du conduit.

Ainsi, grâce à la dernière caractéristique, on va permettre un possible retrait du dispositif médical, et grâce à la première et à la seconde caractéristiques, on va assurer à la fois la souplesse et un encombrement limité des pattes, favorisant ainsi une implantation par une gaine de faible diamètre et une souplesse de manoeuvre du dispositif, tout en assurant une surface d'appui favorable contre la paroi du conduit.

Toujours pour favoriser la souplesse et l'élasticité du dispositif et limiter son encombrement essentiellement radial, la longueur d'allongement du premier tronçon des pattes sera de préférence inférieure à celle du second tronçon.

Dans un souci de favoriser encore le retrait sans nuire à la stabilité dans le cadre d'une implantation "temporaire", une autre caractéristique de l'invention prévoit que la surface aplatie du premier tronçon pourra être recourbée le long d'une direction sensiblement parallèle à l'axe général.

Pour la réalisation industrielle du dispositif, plusieurs solutions ont été retenues :
- une réalisation des premier et second tronçons comme une plaque monobloc (avec diminution progressive de la largeur de cette plaque à partir de l'extrémité libre du premier tronçon vers le second tronçon),
- une réalisation de ces deux tronçons toujours monobloc mais en forme de fil sensiblement rond (pour le second tronçon) puis de plaque (pour le premier tronçon) ou encore, en plusieurs parties avec un premier tronçon sensiblement en plaque et un second tronçon comprenant plusieurs fils.

Après une mise au point difficile, liée aux exigences précitées effectivement difficilement compatibles entre elles, une autre caractéristique de l'invention prévoit que le premier tronçon présente une largeur comprise entre 0,7 mm et 1,5 mm environ, avec une épaisseur comprise entre environ 0,05 mm et 0,25 mm, tandis que la dimension maximale du second tronçon sera de préférence inférieure à environ 0,7 mm.

Dans le cadre déjà cité des filtres sanguins, il est en outre à noter que l'invention est tout particulièrement intéressante dans l'hypothèse d'une utilisation d'abord temporaire puis définitive du filtre à l'intérieur du vaisseau récepteur.

Dans ce domaine, on connaît notamment à partir de EP-A-348 295, un filtre implantable par l'intermédiaire d'une gaine d'implantation, ce filtre comprenant, outre des premiers moyens de filtration à pattes allongées dépourvues de moyens d'accrochage au vaisseau, des seconds moyens de maintien en place de ces moyens de filtration, pour permettre le passage du filtre d'une "utilisation temporaire" à une situation "d'utilisation définitive".

Or, les mêmes inconvénients que ceux déjà mentionnés précédemment existent vis-à-vis de EP-A-348 295.

Et la solution de l'invention est à nouveau, dans ce cas, de prévoir qu'(au moins) une dimension maximale de la surface aplatie essentiellement pleine du premier tronçon des pattes "de filtration" soit supérieure à la dimension maximale du second tronçon, les caractéristiques complémentaires déjà énoncées pouvant bien entendu également être prévues. On y associera alors la qualité de filtration (par un bon appui centré) et un retrait possible.

Il en est de même dans l'hypothèse où le filtre à possible utilisation temporaire ou définitive est du type divulgué dans FR-A-2 718 949 (publication postérieure).

Pour la réalisation industrielle du dispositif médical intraluminal présenté ci-avant, plusieurs solutions ont été retenues.

Selon une première possible réalisation, les tronçons des pattes seront réalisés en une seule pièce, à partir d'un fil allongé de section arrondie, dont une partie sera écrasée pour constituer le premier tronçon.

En alternative, les premier et second tronçons seront réalisés à partir respectivement de un ou plusieurs fils allongés et d'une plaque, et ces fils et la plaque seront ensuite réunis ensemble, par exemple par sertissage ou soudure.

Ce qui suit présente une description plus détaillée de l'invention, à partir des dessins annexés dans lesquels :
- la figure 1 présente une première variante de réalisation montrant à l'état de repos un filtre sanguin intraluminal à utilisation exclusivement temporaire,
- la figure 2 montre un détail du filtre de la figure 1,
- les figures 3 et 4 montrent chacune une alternative de réalisation du segment de patte illustré sur la figure 2,
- la figure 5 montre une possible réalisation d'un filtre à utilisation temporaire/définitive,
- la figure 6 montre un détail d'une patte du filtre de la figure 5,
- et la figure 7 montre, en vue agrandie, une partie du même filtre dans un état radialement replié.

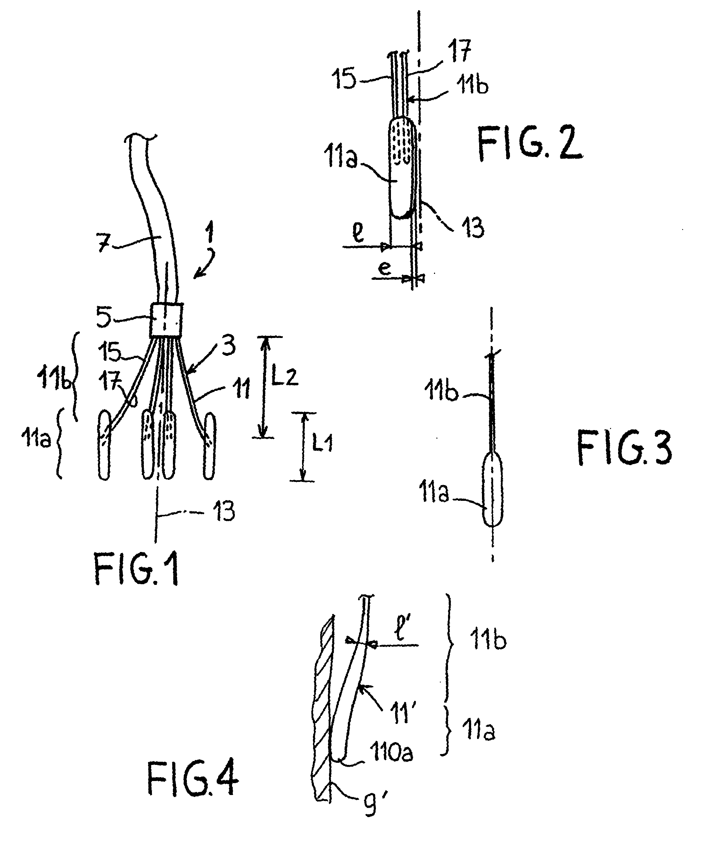

Sur la figure 1 tout d'abord, on voit illustré un appareil de filtration intraluminal 1, pouvant être mis en place par dénudation, ou de préférence par voie percutanée (méthode classique dite de "Seldinger").

L'appareillage 1 comprend le filtre sanguin proprement dit 3 autoexpansible, dont la tête 5 est raccordée à une tige de manoeuvre souple biocompatible 7.

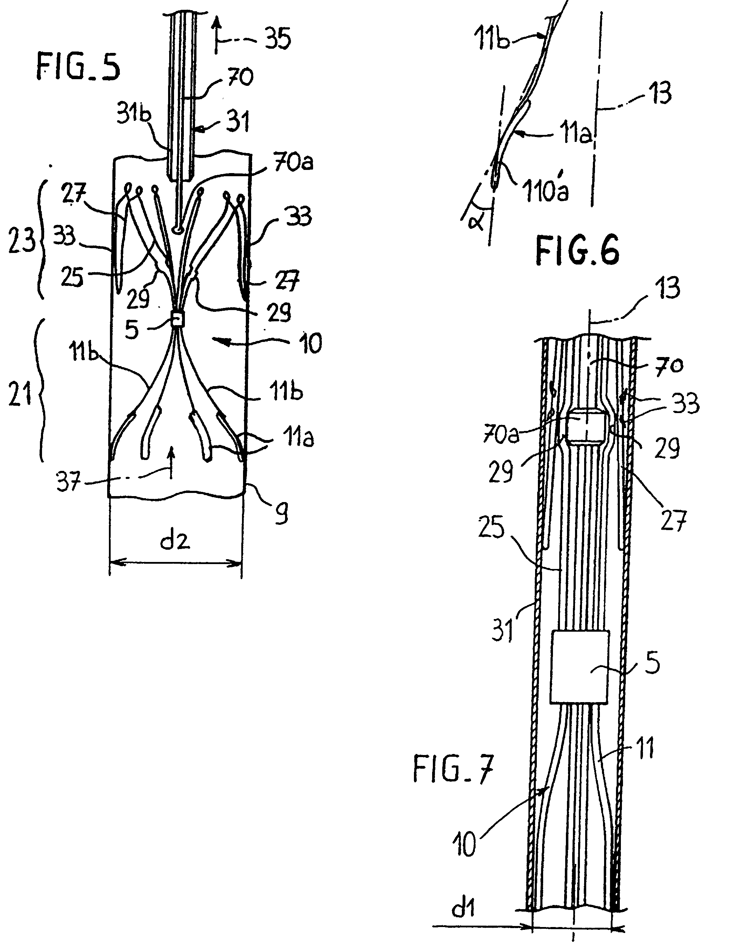

Le filtre 3, qui est dépourvu de moyens d'ancrage à son vaisseau récepteur (qui pourraît être le vaisseau 9 de la figure 5) comprend une structure essentiellement filiforme, c'est-à-dire avec des pattes 11 fines, allongées sensiblement suivant un axe général 13 lorsque le filtre est radialement contraint et que ses pattes sont donc sensiblement parallèles entre elles.

En l'espèce, le filtre se présente sous la forme générale d'une corolle sensiblement conique dans son état illustré radialement non contraint, les pattes 11 étant régulièrement réparties autour de l'axe 13 qui est un axe de révolution. Les pattes 11, dont le nombre est de préférence compris entre 4 et 8, peuvent être considérées comme présentant deux tronçons, un premier tronçon 11a et un second tronçon 11b. Le tronçon 11a, qui termine les pattes du côté de plus grand diamètre de la corolle, permet au filtre de venir en contact du vaisseau tandis que le second tronçon 11b, outre sa fonction de filtration, assure l'ouverture en corolle du filtre ou sa fermeture radiale, en s'étendant plus à l'intérieur du cône lorsque la corolle est déployée, sans donc venir a priori particulièrement en contact du vaisseau.

Tandis que la partie 11a présente une forme aplatie, la partie 11b est plus fine. Plus précisément, on peut voir sur la vue agrandie de la figure 2 que, lorsque le filtre est dans son état radialement contraint permettant son implantation, la dimension maximale l perpendiculairement à l'axe 13 de la surface aplatie 11a est supérieure à celle du tronçon 11b correspondant, de telle sorte que l'encombrement radial sur la partie 11b est inférieure à celle sur la partie 11a.

Toujours sur les figures 1 et 2, on notera encore que sur leur partie terminale 11a, les pattes 11 se présentent sensiblement en plaque pleine à bord périmétrique arrondi, tandis qu'elles se présentent en fil rond sur leur longueur 11b, étant précisé qu'avantageusement, la longueur L₁ du tronçon 11a sera inférieure à la longueur L₂ du tronçon 11b, suivant l'axe 13. Et la partie 11a en plaque s'étend sensiblement parallèlement à l'axe 13 (et donc à la paroi du vaisseau), en position tant radialement contrainte du filtre que radialement déployée.

Compte tenu des fonctions qui leurs sont dévolues, les tronçons élargis d'appui 11a s'étendront du côté de l'extrémité libre de plus grand diamètre d'ouverture du filtre, tandis que les tronçons 11b s'étendront suivant un plus petit diamètre, jusqu'à ce que, dans le cas de l'illustration de la figure 1, les deux fils ronds 15, 17, qui forment le tronçon 11b, soient réunis ensemble à l'endroit de la tête de maintien 5.

Une délicate mise au point a montré qu'il est préférable que la largeur l du "tronçon d'appui" 11a soit comprise entre 0,8 mm et 1,2 mm, avec une épaisseur e comprise entre 0,1 mm et 0,2 mm, la dimension maximale "transversale" des fils 15, 17, du second tronçon 11b (en l'espèce, le diamètre) étant de préférence comprise entre 0,2 mm et 0,5 mm, par exemple de 0,35 mm, ceci pour une réalisation en métal, par exemple en Phynox (marque déposée), ou en tout acier inoxydable approprié.

Dans l'hypothèse de réalisation des figures 1 et 2, les fils 15, 17, et la plaque 11a constitueront des pièces distinctes, les fils (qui s'étendront de préférence sensiblement parallèlement l'un à l'autre) pouvant être sertis, ou de préférence soudés, à la plaque.

Mais dans une variante de réalisation telle qu'illustrée sur la figure 3, on pourrait prévoir de réaliser chaque patte 11 avec un tronçon 11b en fil rond unique, prolongé de façon monobloc par le tronçon 11a, suivant la direction générale d'allongement de la patte.

Pour obtenir que le tronçon 11a soit plus aplati que le tronçon 11b, on pourra procéder par écrasement local du fil métallique utilisé, par exemple par laminage, ou au contraire par étirage, voire par électrolyse.

A noter toutefois que la réalisation des figures 1, 2 et 3 montre un élargissement assez brusque des pattes entre les tronçons 11b et 11a, même si les pattes ainsi constituées peuvent toujours être considérées comme "filiformes".

Au contraire, la patte filiforme 11' illustrée sur la figure 4 présente une largeur l' qui va en augmentant progressivement le long d'abord du second tronçon 11b, puis du premier tronçon terminal 11a que l'on a en l'espèce figuré en appui contre une paroi de conduit 9'. Pour obtenir une telle forme de pattes "à élargissement progressif", on peut là encore recourir à une technique de laminage ou d'étirage, voire d'électrolyse, avec par exemple, une épaisseur allant en diminuant au fur et à mesure que l'on se rapproche de l'extrémité libre 110a du tronçon 11a.

Sur les figures 5 et 7 maintenant, a été représenté l'essentiel d'une structure de filtration pouvant être utilisée de façon temporaire ou définitive.

Sur la figure 5 tout d'abord, on retrouve, dans une constitution structurelle un peu différente, le filtre proprement dit 10 et sa tige de manoeuvre flexible 70.

Si la partie principale de filtration sanguine du filtre 10 ressemble assez étroitement à celle du filtre de la figure 1, avec des pattes en fil rond (en l'espèce simple) prolongées sensiblement axialement par de petites plaques pleines allongées 11a, et se développant d'une manière générale suivant une corolle conique, à partir d'une tête 5 de raccordement des différents tronçons proximaux 11b, on peut constater qu'à l'opposé de cette partie filtrante frontale 21, le filtre présente une deuxième corolle conique 23, les deux corolles étant disposées tête-bêche et réunies par la tête 5.

La seconde corolle 23 a pour fonction principale d'assurer un maintien du filtre en implantation définitive de celui-ci. Elle est réalisée en fil(s) présentant encore deux tronçons, un premier tronçon 25 se développant sensiblement en cône à partir de la tête 5, puis un second tronçon 27 en épingle à cheveux, définissant des sortes de patins de centrage s'étendant sensiblement parallèlement à la paroi 9, dans l'état implanté du filtre.

Pour son raccordement à la tige de manoeuvre 70, le filtre 10 qui est en l'espèce séparable de cette tige, présente en outre des créneaux 29 formés par déformation locale des tronçons 25, le creux de ces déformations recevant une tête élargie 70a formée à l'extrémité de la tige 70, lorsque le filtre 10 est radialement contraint à l'intérieur de la gaine souple d'introduction 31, comme cela est illustré sur la figure 7.

Sur la vue agrandie de la figure 6, le tronçon aplati 11a présente une forme courbée vers l'intérieur de la corolle pour suivre une direction sensiblement parallèle à l'axe général 13 lorsque, comme cela a été supposé, le filtre (et donc ses pattes 11) sont dans l'état radialement contraint. L'angle de courbure, α, sera de préférence compris entre environ 0° et 45°. C'est alors essentiellement la partie terminale libre 110'a du tronçon 11a qui viendra en appui contre la paroi 9.

Sur la figure 7, on retrouve le filtre 10 avec ses pattes 11 ; 25, 27, en double corolle tête-bêche, chacune radialement contrainte suivant l'axe principal 13 qui est également ici l'axe de la gaine ou cathéter d'implantation 31 à l'intérieur duquel le filtre est contenu, avec alors un diamètre d₁ (correspondant au diamètre intérieur du cathéter) inférieur (à l'échelle près) à celui, d₂, qu'il présente lorsqu'il est dans un état implanté à l'intérieur du vaisseau 9 (voir figure 5).

A noter, sur la figure 7, que dans cet état du filtre prêt à être implanté, les créneaux 29 reçoivent la tête 70a de la tige 70, ce qui permet alors de manoeuvrer le filtre depuis l'extérieur du corps, suivant l'axe 13, dans la mesure où (de manière classique) tant la gaine 31 que la tige 70 ont une longueur axialement suffisante pour s'étendre entre l'extérieur du corps du patient considéré et le vaisseau d'implantation (qui peut en particulier être la veine cave).

A noter également, comme on le voit sur la figure 5 et plus nettement sur la figure 7, que les appendices 27 comportent des moyens de fixation 33, en l'espèce en forme de doubles crochets inversés qui vont permettre l'ancrage du filtre dans la paroi du vaisseau, lors de l'implantation définitive.

La technique d'implantation d'un filtre, qu'il soit pour une utilisation temporaire ou définitive, est indiquée dans les publications précitées, ou encore dans US-A-4 990 156.

De manière générale, après avoir ménagé une incision à travers la peau du patient, on forme une voie d'accès jusqu'à la zone vasculaire d'implantation, puis on y glisse la gaine introductrice 31 pour qu'elle s'étende le long de la voie d'accès, son extrémité proximale ressortant du corps, tandis que son extrémité distale atteint le vaisseau d'implantation. Après avoir été radialement contraint à l'intérieur de la gaine, devant la tige de manoeuvre, le filtre est ensuite poussé par cette tige le long du cathéter, jusqu'à l'extrémité distale de la gaine (repérée 31b sur la figure 5). Lorsque cette extrémité est correctement positionnée à l'intérieur du vaisseau, un déplacement de la gaine par rapport à la tige permet de faire sortir le filtre dont les pattes s'expansent naturellement en corolle.

Avec le filtre des figures 5 et 7, on peut dans un premier temps ne procéder qu'à la sortie des pattes 11, les pattes de maintien avec leurs crochets 33 étant retenues à l'intérieur de la gaine où il y a toujours coopération entre les créneaux 29 et la tête 70a de la tige, permettant encore une manoeuvre d'avance ou de recul du filtre.

Du fait que, dans cette position, seuls les premiers tronçons 11a (qui sont dépourvus de crochets d'ancrage) viennent au contact de la paroi du vaisseau (et ce sur une surface d'appui essentiellement pleine et assez importante), il est possible au bout d'une à quelques semaines de retirer le filtre, par traction arrière de celui-ci via la tige 70, ou avancée de la gaine.

Si, par contre, au bout de cette première période d'implantation, le praticien juge que les risques d'embolie demeurent, il peut procéder à une implantation définitive du filtre. Pour cela, il tire la gaine 31 à l'arrière (flèche 35) en maintenant le filtre en position, de telle sorte que la seconde corolle 23 s'expanse, les appendices 27 et leurs crochets 33 venant alors respectivement se plaquer et s'ancrer dans la paroi du vaisseau (figure 5). Une fois le filtre ainsi implanté (avec bien entendu sa première corolle 21 disposée face au flux sanguin 37), le praticien retire la gaine et la tige (alors bien entendu déjà séparée du filtre comme le montre la figure 5), ceci par la voie d'accès déjà utilisée, le filtre étant laissé à demeure dans le vaisseau.

## Revendications

1. Ensemble médical adapté pour être introduit dans un conduit anatomique du corps d'un patient, comprenant :
- un dispositif intraluminal adapté pour être implanté dans le conduit et pour en être retirable dans un état déterminé, et comportant une structure essentiellement filiforme, présentant un axe principal (13), cette structure étant adaptée pour occuper un premier diamètre réduit dans un état radialement replié du dispositif permettant ainsi son implantation, et un second diamètre élargi, dans un état implanté dudit dispositif, et comprenant des pattes (11) allongées présentant, dans ledit état implanté, un premier tronçon (11a) propre à venir au contact d'une paroi du conduit, et un second tronçon (11b),
- des moyens d'implantation ou de retrait du dispositif, comprenant:
• une gaine (31) dans laquelle certaines au moins des pattes (11) du dispositif sont radialement repliées pour son implantation dans le conduit, ou son retrait hors de ce conduit, et de laquelle ledit dispositif peut sortir pour une expansion radiale dans le conduit,
• et une tige (7,70) de manoeuvre adaptée pour coulisser dans la gaine et présentant, avec le dispositif, des moyens de coopération (5 ; 29,70a) pour pousser ou tirer ce dispositif par rapport à la gaine ou au conduit, lors de son implantation ou lors de son retrait, la gaine et la tige présentant une longueur axiale suffisante pour s'étendre entre l'extérieur du corps du patient et l'endroit où le dispositif est implanté dans le conduit,
**caractérisé en ce que** :
-- le premier tronçon (11a) de certaines au moins des pattes présente une surface aplatie essentiellement pleine d'appui contre la paroi du conduit,
-- sensiblement perpendiculairement à l'axe principal (13), et dans l'état radialement replié du dispositif, ledit premier tronçon présente une dimension maximale supérieure à celle du second tronçon (11b), et
-- le premier tronçon (11a) est dépourvu de moyens d'accrochage à la paroi du conduit.

2. Dispositif selon la revendication 1, **caractérisé en ce que** le premier tronçon (11a) a la forme sensiblement d'une plaque et le second tronçon (11b) comprend au moins un fil de section arrondie.

3. Dispositif selon la revendication 1 ou la revendication 2, **caractérisé en ce que** la longueur d'allongement (L1) du premier tronçon des pattes est inférieure à celle (L2) du second tronçon.

4. Dispositif selon l'une quelconque des revendications précédentes, **caractérisé en ce que** la surface aplatie du premier tronçon (11a) est courbée le long d'une direction sensiblement parallèle audit axe principal (13).

5. Dispositif selon l'une quelconque des revendications précédentes, **caractérisé en ce que** les premier et second tronçons (11a, 11b) sont monoblocs.

6. Dispositif selon l'une quelconque des revendications 1 à 4, **caractérisé en ce que** le second tronçon (11b) comprend plusieurs fils de section arrondie (15, 17).

7. Dispositif selon l'une quelconque des revendications 1, 3, 4 ou 5, **caractérisé en ce que** les premier et second tronçons ont la forme d'une plaque monobloc.

8. Dispositif selon la revendication 7, **caractérisé en ce que** le premier tronçon des pattes présente une extrémité libre (110a) et ladite plaque monobloc présente, suivant la direction d'allongement de la patte considérée, une longueur et, perpendiculairement à cette direction, une largeur (l') et une épaisseur, ladite largeur augmentant depuis le second tronçon en direction de l'extrémité libre du premier tronçon.

9. Dispositif selon l'une quelconque des revendications précédentes, **caractérisé en ce que** le premier tronçon (11a) présente une largeur comprise entre environ 0,7 mm et 1,5 mm et une épaisseur comprise entre environ 0,05 mm et 0,25 mm.

10. Dispositif selon l'une quelconque des revendications précédentes, **caractérisé en ce que** ladite dimension maximale du second tronçon (11b) est inférieure à 0,7 mm environ.

## Patentansprüche

1. Medizinische Konstruktion, die in ein anatomisches Gefäß des Körpers eines Patienten eingeführt werden kann, bestehend aus:
- einer intraluminalen Vorrichtung, die in das Gefäß eingepflanzt werden kann und aus diesem in einem bestimmten Zustand herausgezogen werden kann und die eine im Wesentlichen drahtförmige Struktur besitzt, die eine Hauptachse (13) aufweist, wobei diese Struktur in einem radial zusammengefalteten Zustand der Vorrichtung einen ersten verringerten Umfang annehmen kann, der deren Einpflanzung erlaubt, und die in einem eingepflanzten Zustand der Vorrichtung einen zweiten, vergrößerten Umfang annehmen kann und langgestreckte Klammern (11) besitzt, die in dem eingepflanzten Zustand einen ersten Abschnitt (11a) bieten, der mit der Wand des Gefäßes zum Anliegen kommen kann, sowie einen zweiten Abschnitt (11b),
- wobei die Mittel zum Einpflanzen oder zum Herausziehen der Vorrichtung bestehen aus:
· einer Hülse (31) in der wenigstens einige Klammern (11) der Vorrichtung zum Einpflanzen in das Gefäß oder zum Herausziehen aus dem Gefäß radial zusammengefaltet sind und aus der die Vorrichtung für eine radiale Expansion in dem Gefäß austreten kann.
· und einem Betätigungsstift (7, 70) der in der Hülse gleiten kann und Mittel zum Zusammenwirken (5; 29, 70a) mit der Vorrichtung aufweist, um die Vorrichtung gegenüber der Hülse oder dem Gefäß während ihrer Einpflanzung oder ihres Rückzugs zu schieben oder zu ziehen, wobei die Hülse und der Stift eine Längsachse aufweisen, die ausreichend ist, um sich zwischen dem Äußeren des Körpers des Patienten und dem Ort zu erstrecken, an dem die Vorrichtung in das Gefäß eingepflanzt wird,
**dadurch gekennzeichnet, dass**:
- der erste Abschnitt (11a) wenigstens einiger Klammern eine abgeflachte, im Wesentlichen solide Stützfläche zu der Wand des Gefäßes aufweist,
- der erste Abschnitt im Wesentlichen senkrecht zur Hauptachse (13) und im zusammengefalteten Zustand der Vorrichtung eine größere Dimension aufweist, als der zweite Abschnitt (11b), und
- der erste Abschnitt (11a) frei von Mitteln zur Haftung an der Wand des Gefäßes ist.

2. Vorrichtung nach Anspruch 1, **dadurch gekennzeichnet, dass** der erste Abschnitt (111a) im Wesentlichen die Form einer Polatte besitzt und der zweite Abschnitt (11b) aus wenigstens einem im Querschnitt runden Draht besteht.

3. Vorrichtung nach Anspruch 1 oder 2, **dadurch gekennzeichnet, dass** die Längsausdehnung (L1) des ersten Abschnitts geringer ist, als die des zweiten Abschnitts (L2).

4. Vorrichtung nach einem der vorhergehenden Ansprüche, **dadurch gekennzeichnet, dass** die abgeflachte Oberfläche des ersten Abschnitts (11a) entlang einer im Wesentlichen parallel zu der Hauptachse (13) liegenden Richtung gewölbt ist.

5. Vorrichtung nach einem der vorhergehenden Ansprüche, **dadurch gekennzeichnet dass** die ersten und die zweiten Abschnitte (11a, 11b) einstückig sind.

6. Vorrichtung nach einem der Ansprüche 1 bis 4, **dadurch gekennzeichnet, dass** der zweite Abschnitt aus mehreren Drähten mit rundem Querschnitt besteht (15, 17).

7. Vorrichtung nach einem der Ansprüche 1, 3, 4 oder 5, **dadurch gekennzeichnet dass** der erste und der zweite Abschnitt die Form einer einstückigen Platte aufweisen.

8. Vorrichtung nach Anspruch 7, **dadurch gekennzeichnet, dass** der erste Abschnitt der Klammern ein freies Ende (110a) besitzt und die einstückige Platte in Richtung der Ausdehnung der betrachteten Klammer ein Breite (l') und eine Stärke aufweisen, wobei die Breite von dem zweiten Abschnitt zu dem freien Ende des ersten Anschnitts zunimmt.

9. Vorrichtung nach einem der vorhergehenden Ansprüche, **dadurch gekennzeichnet, dass** der erste Abschnitt (11a) eine Breite von zwischen etwa 0,7 mm und 1,5 mm und eine Stärke von zwischen etwa 0,05 mm und 0,25 mm aufweist.

10. Vorrichtung nach einem der vorhergehenden Ansprüche, **dadurch gekennzeichnet, dass** die genannte maximale Dimension des zweiten Abschnitts (11b) unter etwa 0,7 mm liegt.

## Claims

1. A medical intraluminal assembly intended to be introduced in an anatomical passage of a patient body, comprising :
- an intraluminal device to be implanted in the passage and to be extracted from it in a determined state, and having a substantially filiform structure, having a main axis (13), such a structure being adapted to occupy a first reduced diameter in a radially restrained state of the device allowing it to be implanted, and a second widened diameter, in an implanted state of the said device, and comprising elongated legs (11) having, in said implanted state, a first portion (11a) adapted to contact a wall of said passage, in said implanted state and a second portion (11b),
- device implantation or extraction means, including :
* a sheath (31) in which at least some of legs (11) of the device are radially bent for implantation in the passage, ou for extraction from said passage, and from which said device may exit to be radially expanded in the passage, and
* an actuation rod (7, 70) for sliding in the sheath and having, with the device, engaging means (5 ; 29, 70a) for pushing or drawing the device in relation to the sheath or the passage, during implantation or extraction, the sheath and the rod having an axial length sufficient for extending between outside the patient body and the place of implantation of the device in the passage,
**characterized in that** :
- the first portion (11a) of at least some of the legs has a substantially solid flattened surface, for bearing on the wall of the passage,
- substantially at right-angles to the main axis (13) and in the radially restrained state of the device, said first portion has a maximum dimension which is greater than that of the second portion (11b), and
- the first portion (11a) is devoid of means of fixation to the wall of the passage.

2. A device according to claim 1, in which the first portion (11a) substantially takes the form of a plate while the second portion (11b) comprises at least one filament of round cross-section.

3. A device according to claim 1 or claim 2, in which the elongation length (L1) of the first portion of the legs is less than that (L2) of the second portion.

4. A device according to any preceding claim, in which the flattened surface of the first portion (11a) is curved along a direction substantially parallel with the said main axis (13).

5. A device according to any preceding claim, in which the first and second portions (11a, 11b) are integral with one another.

6. A device according to any of claims 1 to 4, in which the second portion (11b) comprises a plurality of filaments (15, 17) of rounded cross-section.

7. A device accordingly to any of claims 1, 3, 4 or 5, in which the first and second portions take the form of an integral plate.

8. A device accordingly to claim 7 in which the first portion of the legs has a free end (110a) and the said integral plate has, according to the direction of elongation of one of said legs, a length and, at right-angles to said direction, a width (l') and a thickness, the said width increasing from the second portion in the direction of the free end of the first portion.

9. A device according to claim 1 in which the first portion (11a) has a width between approximately 0.7 mm and 1.5 mm and a thickness between approximately 0.05 mm and 0.25 mm.

10. A device according to claim 1 in which the said maximum dimension of the second portion (11b) is less than approximately 0.7 mm.
